(19) Europäisches Patentamt
European Patent Office
Office européen des brevets

(11) Numéro de publication : **0 115 466**
**B1**

(12) # FASCICULE DE BREVET EUROPÉEN

(45) Date de publication du fascicule du brevet : **16.06.87**

(21) Numéro de dépôt : **84420008.9**

(22) Date de dépôt : **25.01.84**

(51) Int. Cl.[4] : **C 07 F 9/48**, C 07 F 9/142, C 07 C149/46, C 07 C161/00, A 01 N 57/18, A 01 N 57/10, A 01 N 31/00, A 01 N 41/00

(54) **Sels de dérivés organophosphorés fongicides.**

(30) Priorité : **01.02.83 FR 8301727**
**13.10.83 FR 8316499**

(43) Date de publication de la demande :
**08.08.84 Bulletin 84/32**

(45) Mention de la délivrance du brevet :
**16.06.87 Bulletin 87/25**

(84) Etats contractants désignés :
**AT BE CH DE FR GB IT LI LU NL SE**

(56) Documents cités :
**EP-A- 0 031 054**
**EP-A- 0 038 778**
**EP-A- 0 053 871**
**DE-A- 2 617 743**
**FR-A- 2 317 286**
**FR-A- 2 457 873**

(73) Titulaire : **RHONE-POULENC AGROCHIMIE**
**14-20, rue Pierre Baizet**
**F-69009 Lyon (FR)**

(72) Inventeur : **Lacroix, Guy**
**332F Balmont-La Duchère**
**F-69009 Lyon (FR)**
Inventeur : **Anding, Claude**
**Domaine de la Source No. 2**
**F-69630 Chaponost (FR)**
Inventeur : **Viricel, Andrée**
**39, Rue Clément Michut**
**F-69100 Villeurbanne (FR)**

(74) Mandataire : **Chrétien, François et al**
**RHONE-POULENC AGROCHIMIE BP 9163**
**F-69263 Lyon Cedex 09 (FR)**

**EP 0 115 466 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention concerne de nouveaux sels de dérivés organophosphorés, la préparation de ces composés ainsi que les compositions fongicides les contenant et leur mode d'application pour la protection des plantes.

La demande FR-A-2 317 286 décrit des sels de sulfonium très particuliers, à savoir des alcoylsulfates d'hydroxy-2 éthyl alcoyl alcoyl sulfonium, comme fongicides utilisables sur plantes. Cependant, ces composés ne contiennent pas d'anion phosphoré, ont un spectre d'activité limité et présentent des risques importants de phytotoxicité illustrés aux exemples 5-10.

La demande EP-A 0 053 871 concerne des composés ayant un anion phosphoré et un cation sulfonium mais qui sont des sels de sulfonium de la N-phosphonométhyl glycine à haute activité herbicide, qui interdit toute utilisation pour la protection des plantes contre les maladies, qui suppose bien entendu le respect desdites plantes.

Les demandes EP-A 00 31 054 et EP-A 00 38 778 décrivent des compositions fongicides à base de composés ayant un anion phosphoré mais dont le cation, quand il est organique, comporte nécessairement au moins un atome d'azote portant la charge positive.

Les nouveaux composés selon l'invention répondent à la formule générale :

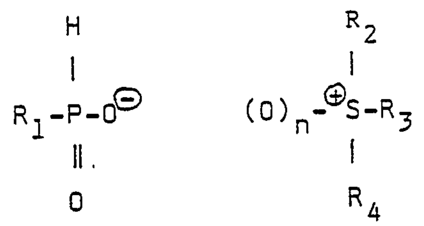

(I)

dans laquelle :

$R_1$ représente un atome d'hydrogène, un groupe hydroxyle, un radical alcoyle inférieur contenant de 1 à 4 atomes de carbone, éventuellement substitué par un atome d'halogène, un groupe hydroxyle ou hydroxylamino, ou représente un phényle, ou encore un radical $OR_5$ dans lequel $R_5$ représente un alcoyle contenant de 1 à 4 atomes de carbone,

$R_2$ et $R_3$, identiques ou différents, peuvent représenter un radical alcoyle contenant de 1 à 5 atomes de carbone, ou un phényle, $R_2$ et $R_3$ pouvant en outre former un radical $-(CH_2)_m-$, m étant un nombre entier égal à 4 ou 5.

$R_4$ représente un radical alcoyle contenant de 1 à 18 atomes de carbone, un radical alcényle contenant de 2 à 18 atomes de carbone, le radical benzyle ou phényle,

n est un nombre entier égal à zéro ou 1.

Les composés préférés en raison de leurs propriétés fongicides sont ceux dans la formule desquelles $R_1$ est un atome d'hydrogène, un radical hydroxyle ou alcoyle de 1 à 4 atomes de carbone ou alcoxyle de 1 à 4 atomes de carbone, $R_2$ et $R_3$ sont chacun un radical alcoyle de 1 à 4 atomes de carbone notamment méthyle et éthyle, et $R_4$ est le radical méthyle ou un alcoyle linéaire de 12 à 16 atomes de carbone.

Les composés de formule générale I peuvent être préparés selon un procédé qui consiste à faire réagir, en milieu aqueux un composé de formule :

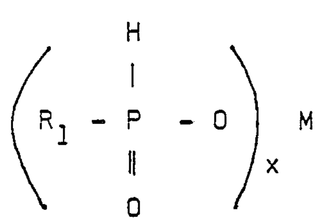

avec un sel de sulfonium ou de sulfoxonium selon le schéma :

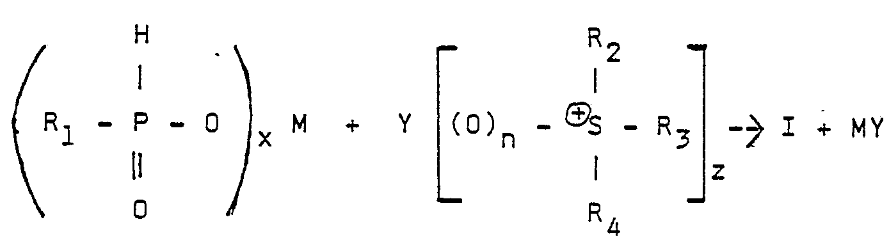

dans lequel $R_1$, $R_2$, $R_3$ et $R_4$ et n ont la même signification que dans la formule I, M est :

— soit un atome d'hydrogène, auquel cas Y est un atome d'halogène et x et z sont égaux à 1,

— soit un atome de métal alcalino-terreux, auquel cas Y est l'anion sulfate et x et z sont égaux à 2.

Ce procédé peut être effectué selon deux variantes :

Une première variante consiste à faire réagir, en milieu aqueux, un acide de formule :

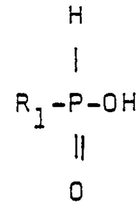

avec un halogénure de sulfonium ou de sulfoxonium selon le schéma :

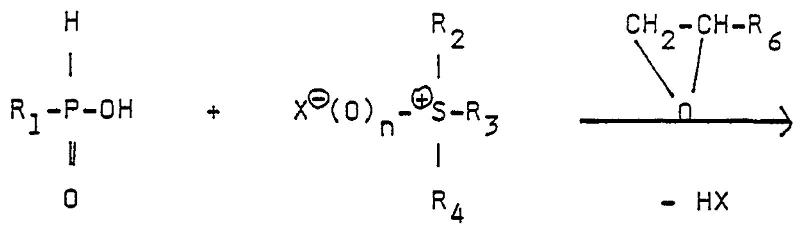


(I)

dans lequel :

$R_1$, $R_2$, $R_3$, $R_4$ et n ont les mêmes significations que dans la formule I,

$R_6$ est un atome d'hydrogène ou un radical méthyle

et X est un atome d'halogène, chlore, brome, iode ou fluor,

en présence d'un accepteur d'hydracide tel que par exemple un oxyde d'alcoylène comme l'oxyde d'éthylène ou de propylène.

Dans le cas des phosphites monosubstitués, (c'est-à-dire lorsque $R_1 = OR_5$), l'ester d'acide phosphoreux est préparé in situ par hydrolyse du dialcoyl phosphite correspondant selon la réaction :

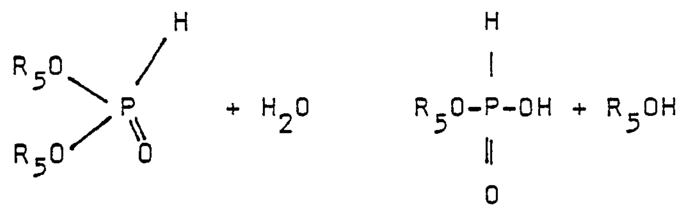

Une seconde variante consiste à faire réagir un sulfate de sulfonium ou de sulfoxonium et un phosphite substitué d'un métal M donnant un sulfate insoluble, notamment un métal acalino-terreux tel que calcium ou baryum, selon le schéma :

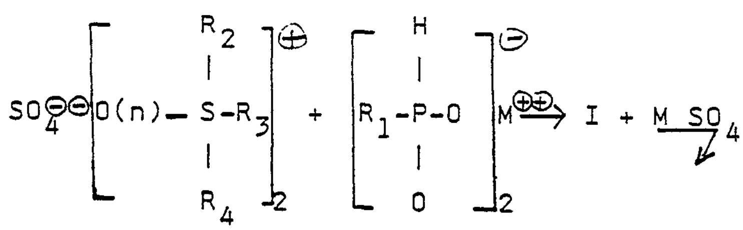

dans lequel $R_1$, $R_2$, $R_3$, $R_4$ et n ont les significations précédentes. Le sulfate insoluble précipite : il est filtré ; le filtrat est concentré et purifié le cas échéant.

Les exemples suivants illustrent la préparation des composés selon l'invention ainsi que leurs propriétés fongicides. La structure des composés a été confirmée par spectrométrie de résonance magnétique nucléaire (RMN) les spectres RMN ont été obtenus avec un spectromètre à 60 Méga Herz.

Dans les exemples illus ant les propriétés biologiques, on considère qu'un produit exerce une protection totale vis-à-vis d'u e maladie fongique lorsque la protection est d'au moins 95 %; la protection est considérée comme bonn lorsqu'elle est d'au moins 80 % (mais inférieure à 95 %).

Exemple 1

Alcoyl et phénylphosphinates de sulfo(xo)nium

On dissout 3,8 g d'acide monoéthylphosphinique et 8,8 g d'iodure de triméthylsulfoxonium dans 80 ml d'eau. On ajoute 20 ml d'oxyde de propylène. On chauffe ensuite le milieu pendant 3 heures à 35 °C. Le milieu est concentré sous pression réduite « 20-26, 7 mbar » (15-20 mm Hg) : on obtient une huile incolore qui cristallise à température ambiante. Le produit brut est dissous dans 50 ml d'acétonitrile à 40 °C. On refroidit la solution dans un bain acétone-carboglace : le produit précipite. Le précipité est filtré et lavé avec 10 ml d'acétonitrile glacé et avec 10 ml d'éther. Le produit est séché dans un dessiccateur sous vide.

On obtient ainsi 4 g d'un solide blanc hygroscopique fondant à 118 °C correspondant à l'éthylphosphinate de triméthylsulfoxonium (composé n° 1).

En opérant selon le même mode opératoire, à partir de l'acide phénylphosphinique et d'iodure de triméthylsulfoxonium, on obtient le phénylphosphinate de triméthylsulfoxonium (composé n° 11), de point de fusion égal à 124 °C.

En procédant selon le même mode opératoire, à partir de l'acide phosphinique et de l'iodure de méthyldialcoyl sulfonium appropriés, on obtient les composés suivants :

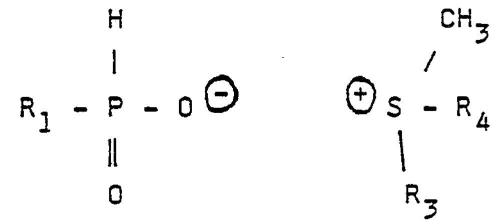

| Composé n° | $R_1$ | $R_3$ | $R_4$ | Constantes Physiques |
|---|---|---|---|---|
| 2 | $C_2H_5$ | $CH_3$ | $CH_3$ | Solide blanc très hygroscopique |
| 12 | $C_6H_5$ | $CH_3$ | $CH_3$ | PF = 135°C |
| 13 | $C_2H_5$ | $CH_3$ | $C_{12}H_{25}$ | PF = 96°C |
| 14 | $C_6H_5$ | $CH_3$ | $C_{12}H_{25}$ | PF = 93°C |
| 15 | $C_2H_5$ | $CH_3$ | $C_{13}H_{27}$ | PF = 90°C |
| 16 | $C_6H_5$ | $CH_3$ | $C_{13}H_{27}$ | PF = 85°C |
| 17 | $C_2H_5$ | $CH_3$ | $C_{14}H_{29}$ | PF = 93-95°C |
| 18 | $C_6H_5$ | $CH_3$ | $C_{14}H_{29}$ | PF = 80-85°C |
| 19 | $C_2H_5$ | $CH_3$ | $C_{16}H_{33}$ | PF =95-100°C |
| 20 | $C_6H_5$ | $CH_3$ | $C_{16}H_{33}$ | PF = 92-95°C |
| 33 | $C_2H_5$ | $C_2H_5$ | $C_{12}H_{25}$ | PF = 65°C (vitrification) |
| 34 | H-O-CH<br>$CH_3$ | $C_2H_5$ | $C_{12}H_{25}$ | PF = 82-85°C |
| 35 | $C_6H_5$ | $C_2H_5$ | $C_{12}H_{25}$ | Solide pâteux hygroscopique |

Exemple 2

Préparation de l'éthylphosphite de triméthylsulfonium (composé n° 3)

On dissout 5,5 g de diéthylphosphite et 8,2 g d'iodure de triméthylsulfonium dans 80 ml d'eau. On

ajoute 20 ml d'oxyde de propylène puis on chauffe pendant 6 heures à 35 °C. Après concentration du milieu sous pression réduite, on obtient 7,9 g d'une huile incolore qui cristallise à température ambiante. On dissout le produit brut dans 25 ml d'acétonitrile à température ambiante. On refroidit la solution à l'aide d'un bain acétone-glace. Le produit précipité est filtré, puis séché en dessiccateur sous vide.

On obtient ainsi 4 g d'un solide blanc très hygroscopique fondant à 58 °C.

En opérant de la même manière à partir des réactifs appropriés, on a préparé les composés suivants :

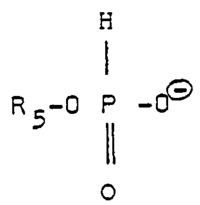 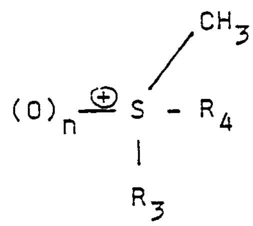

| Composé n° | $R_5$ | n | $R_3$ | $R_4$ | Constante Physique |
|---|---|---|---|---|---|
| 4 | H | 0 | $CH_3$ | $CH_3$ | PF : 125°C |
| 5 | H | 1 | $CH_3$ | $CH_3$ | PF : 173°C |
| 6 | $CH_3$ | 0 | $CH_3$ | $CH_3$ | huile $n_D^{20}$ 1,488 |
| 7 | $CH_3$ | 1 | $CH_3$ | $CH_3$ | PF : 41°C |
| 8 | $C_2H_5$ | 1 | $CH_3$ | $CH_3$ | PF : 78°C |
| 21 | H | 0 | $CH_3$ | $C_{12}H_{25}$ | PF : 65°C |
| 22 | $C_2H_5$ | 0 | $CH_3$ | $C_{12}H_{25}$ | PF: 80°C |
| 23 | H | 0 | $CH_3$ | $C_{13}H_{27}$ | PF : 65°C |
| 24 | $C_2H_5$ | 0 | $CH_3$ | $C_{13}H_{27}$ | PF: 80°C |
| 25 | H | 0 | $CH_3$ | $C_{14}H_{29}$ | PF : 76°C |
| 26 | $C_2H_5$ | 0 | $CH_3$ | $C_{14}H_{29}$ | PF : 92-93°C |
| 27 | H | 0 | $CH_3$ | $C_{16}H_{33}$ | PF : 80°C |
| 28 | $C_2H_5$ | 0 | $CH_3$ | $C_{16}H_{33}$ | PF: 85°C |
| 36 | $C_2H_5$ | 0 | $C_2H_5$ | $C_{12}H_{25}$ | PF : 65°C |

## Exemple 3

### Préparation de l'hypophosphite de triméthylsulfonium (composé n° 9)

A une solution de 5,12 g d'hypophosphite de calcium dans 50 ml d'eau, on ajoute une solution de 7,5 g de sulfate de triméthylsulfonium et 20 ml d'eau, en maintenant le milieu sous agitation ; le sulfate de calcium précipite. On maintient l'agitation pendant 0,5 heure à température ambiante. Après quoi on filtre le précipité. On concentre le filtrat, reprend l'huile résiduelle avec 100 ml d'acétonitrile et filtre les insolubles. On concentre la solution organique, triture l'huile résultante dans 200 ml d'éther et on obtient des cristaux que l'on disperse dans le solvant. On filtre et on sèche le précipité au dessiccateur sous vide.

Dans ces conditions, on obtient 6 g d'un solide blanc hygroscopique fondant à 115-118 °C.

En opérant de la même manière à partir d'hypophosphite ou d'hydroxy-1-éthyle phosphite de calcium et de sulfate de trialcoyl sulfonium approprié, on obtient les composés suivants :

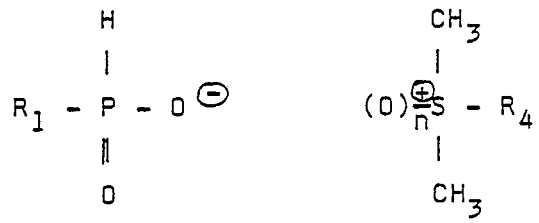

| Composé n° | $R_1$ | n | $R_4$ | Constante Physique |
|---|---|---|---|---|
| 10 | H | 1 | $CH_3$ | PF : 155 °C |
| 29 | H | 0 | $C_{12}H_{25}$ | PF : 50 °C |
| 30 | H | 0 | $C_{16}H_{33}$ | PF : 80-85 °C |
| 31 | $CH_3$<br>\|<br>HO-C-H | 0 | $CH_3$ | PF : 90 °C |
| 32 | $CH_3$<br>\|<br>HO-C-H | 1 | $CH_3$ | PF : 130 °C |

## Exemple 4

Test in vivo sur Plasmopara viticola, responsable du mildiou de la vigne, sur plants de vigne (traitement préventif)

Des plants de vigne (cépage CHARDONNAY), cultivés en pots, sont traités sur les deux faces de leurs feuilles par pulvérisation d'une émulsion aqueuse contenant la matière active à tester ; l'émulsion pulvérisée est constituée de :

40 mg de matière active à tester

40 $cm^3$ d'eau

0,02 $cm^3$ de Tween 80 (marque déposée pour un agent tensioactif constitué d'un oléate de dérivé polyoxyéthylèné du sorbitol).

Cette émulsion, ainsi constituée, permet la pulvérisation d'une émulsion aqueuse contenant 1 g/l de matière active à tester. Pour obtenir des émulsions à pulvériser de concentrations en matière active à tester inférieures à 1 g/l, on dilue à l'eau l'émulsion aqueuse ainsi constituée.

Au bout de 48 heures, la contamination est effectuée par pulvérisation, sur la face inférieure des feuilles, d'une suspension aqueuse de 80 000 unités/$cm^3$ environ de spores du champignon. Ensuite, les pots sont placés pendant 48 heures en cellule d'incubation à 100 % d'humidité relative et à 20 °C.

On effectue le contrôle des plants 9 jours après la contamination.

Dans ces conditions, on constate que :

— à la dose de 1 g/l, les composés 3, 25, 31, 34, assurent une protection totale, et les composés 10, 17, 28, une bonne protection.

— à la dose de 0,33 g/l, les composés 1, 19, 21, 30, assurent une protection totale et les composés 2, 9, 18, 26, 27, une bonne protection.

## Exemple 5

Test in vivo sur « Phytophthora infestans » responsable du mildiou de la tomate

Des plants de tomates (variété Marmande) cultivés en serre et âgés de 60 à 75 jours sont traités par pulvérisation avec des émulsions aqueuses préparées comme indiqué à l'exemple 3 et contenant diverses concentrations de matière active à tester.

Au bout de 48 heures, les plants traités sont contaminés avec une suspension aqueuse de spores (zoosporanges) obtenue à partir d'une culture de « Phytophthora Infestans » cultivée pendant 20 jours sur un milieu à base de farine de pois chiches.

Les plants de tomate sont placés pendant 48 heures dans une enceinte à une température de 16 à 18 °C et ayant une humidité relative de 100 %. L'humidité relative est ensuite ramenée à 80 %.

On observe les résultats 8 jours après contamination. Les résultats s'apprécient par évaluation de la surface de feuilles envahie par le champignon et s'expriment par le « pourcentage de protection » c'est-à-dire 100 [1 — $(S/S_{tm},)$], S étant la surface envahie par le champignon sur le plant considéré et $S_{tm}$ étant la surface envahie par le champignon sur le plant témoin non traité. On indique ci-après les résultats, comme dans les exemples précédents, sous forme de : protection totale, bonne.

Dans ces conditions, on constate que, à la dose de 1 g/l, les composés 4 et 10 assurent une protection totale.

Exemple 6

Test in vivo sur Erysiphe graminis sur orge (oïdium de l'orge)

On prépare par broyage fin une émulsion aqueuse de la matière active à tester ayant la composition suivante :

| | |
|---|---|
| matière active à tester | 40 mg |
| Tween 80 (agent tensioactif constitué d'un oléate de dérivé polyoxyéthylèné du sorbitol) | 0,4 ml |
| eau | 40 ml |

Cette émulsion aqueuse est ensuite diluée par de l'eau pour obtenir la concentration désirée.

De l'orge, en godets, semée dans un mélange de tourbe et de pouzolane, est traitée au stade 10 cm de hauteur par pulvérisation d'une émulsion aqueuse de concentration indiquée ci-après. L'essai est répété deux fois. Au bout de 48 heures, on saupoudre les plants d'orge avec des spores d'Erysiphe graminis, le saupoudrage étant effectué à l'aide de plants malades.

La lecture se fait 10 jours après la contamination.

Dans ces conditions, on constate que, à la dose de 1 g/l, le composé 2 assure une protection totale et les composés 6, 9, 12, une bonne protection.

Exemple 7

Test in vitro sur

— Pythium de Baryanum, responsable de la fonte des semis,

— Botrytis cinerea, responsable de la pourriture grise.

Le composé à tester est ajouté sous forme de solution acétonique (à 1 %) dans un tube à essai contenant un milieu de culture stérile et en surfusion (70 °C). Après mélange, le milieu contenant le produit est coulé aseptiquement dans une boîte de Pétri (10 cm). On réalise ainsi des séries de boîtes contenant diverses doses de matière active. Après 24 heures les boîtes sont inoculées par dépôt au centre d'un implant de mycelium (diamètre 9 mm) du champignon étudié (Pythium) ou d'une goutte de suspension de conidies (Botrytis).

On compare ensuite la vitesse de croissance du champignon sur milieu sans produit (témoin) et sur milieu contenant les doses préalablement décrites ; l'appréciation de la vitesse de croissance des champignons se fait par mesure de diamètre de la colonie.

Dans ces conditions, on constate que, à la dose de 0,1 g/l, les composés 4 inhibent complètement la croissance du Pythium, tandis que les composés 2, 3, 9, 12, 13, 14, 15, 16, 18, 19, 20, 21, 23, 24, 25, 26, 27, 28, 29, 30, assurent une bonne inhibition. De plus à la même dose, les composés 3 et 6 assurent une bonne inhibition de Botrytis cinerea.

Exemple 8

Test in vivo sur Puccinia recondita responsable de la rouille brune des céréales

Des plants de blé (variété TALENT), cultivés en serre, ayant atteint une hauteur de 10 cm environ, sont traités par pulvérisation avec des émulsions aqueuses préparées comme indiqué à l'exemple 3 et contenant diverses concentrations des composés selon l'invention.

Au bout de 48 heures, les plants traités sont contaminés avec une suspension aqueuse de spore de « Puccinia recondita » contenant environ 80 000 spores par ml, préparés à partir de plants déjà contaminés.

Les plants de blé sont placés pendant 48 heures dans une enceinte, à une température de 20 °C environ, et ayant une humidité relative de 100 %. L'humidité relative est ensuite ramenée à 60 %. Le contrôle de l'état des plants se fait le 15ème jour après la contamination et on détermine le pourcentage de protection selon la méthode décrite dans l'exemple 3.

Dans ces conditions, on observe que :

— à la dose de 1 g/l, les composés n° 13, 14, 30, exercent une protection totale, les composés n° 15, 17, 23, 31, 34, une bonne protection,

— à la dose de 0,3 g/l, les composés n° 16, 24, 26, 28, 29 exercent une protection totale,

— à la dose de 0,1 g/l, les composés n° 18, 19, 20, 27 exercent une protection totale.

Test in vitro sur bactéries

On dépose à chaud 20 ml d'un milieu gelosé dans une série de boîte de Pétri de diamètre 9 mm, puis on laisse refroidir le milieu. Simultanément, on injecte dans chaque boîte de Pétri, à l'aide d'une pipette doseuse, une solution à 1 % de la matière à tester dans un solvant organique ou aqueux inerte vis-à-vis de la croissance des bactéries dans les conditions de l'essai.

Au bout de 24 heures, on inocule le contenu des boîtes de Pétri avec la bactérie choisie, puis l'essai est mis en observation dans une pièce à 22 °C ± 2 °C.

Le contrôle est effectué 3 jours après l'inoculation, par comparaison visuelle de la croissance des colonies bactériennes par rapport à un témoin ne contenant pas d'inhibiteur (matière à tester).

Dans ces conditions on observe que :

sur Erwinia amylovora (INRA : CNBP 1430) les composés :
— 11, 12, 15, 16, 19, 22, 23, 24, 27, à la dose de 30 mg/l
— 13, 14, 21, à la dose de 10 mg/l,
inhibent totalement la croissance de la bactérie,

sur Xanthomonas oryzae (INRA : CNBP 1951) les composés :
— 27, à la dose de 30 mg/l,
— 22, à la dose de 10 mg/l,
— 13, 14, 16, 17, 18, 21, 24, 25, 26, 28, à la dose de 3 mg/l,
— 11, 12, 15, 19, 23 à la dose de 1 mg/l,
inhibent totalement la croissance de la bactérie.

sur Corynebacterium michiganense (INRA : CNBP 2108) les composés :
— 11, 12, 19, 27 à la dose de 10 mg/l,
— 13, 14, 15, 16, 17, 18, 21, 22, 23, 24, 26, à la dose de 3 mg/l,
— 25, 28, à la dose de 1 mg/l,
inhibent totalement la croissance de la bactérie.

Les exemples précédents illustrent clairement les remarquables propriétés fongicides des compositions à base des composés selon l'invention sur des familles de champignons variées comme notamment les phycomycètes tels que Plasmapora viticola, les Phytophthora et les Pythium, ou encore les Ascomycètes tels que Erysiphe sp (oidiums) ou botrytis, ou les basidiomycètes tels que Puccinia recondita ou encore les Fungi imperfecti tels que Piricularia oryzae. Il faut noter également l'excellente action bactéricide des composés selon l'invention sur d'importantes bactéries agricoles telles que celles du type Erwinia, du type Xanthomonas et autres Corynebacterium sp.

Ils présentent enfin une bonne sélectivité vis-à-vis des cultures.

Ils s'appliquent avantageusement à des doses de 0,05 à 5 kg/ha, de préférence de 0,1 à 2 kg/ha.

Pour leur emploi pratique, les composés selon l'invention sont rarement utilisés seuls. Le plus souvent ils font partie de compositions. Ces compositions, utilisables pour la protection des végétaux contre les maladies fongiques, contiennent comme matière active un composé selon l'invention tel que décrit précédemment en association avec les supports solides ou liquides, acceptables en agriculture et les agents tensio-actifs également acceptables en agriculture. En particulier sont utilisables les supports inertes et usuels et les agents tensio-actifs usuels.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Ces doses d'emploi comme fongicides des composés selon l'invention peuvent varier dans de larges limites, notamment selon la virulence des champignons et les conditions climatiques.

D'une manière générale des compositions contenant 0,5 à 5 000 ppm de substance active conviennent bien, ces valeurs sont indiquées pour les compositions prêtes à l'application. « Ppm » signifie « partie par million ». La zone de 0,5 à 5 000 ppm correspond à une zone de $5 \times 10^{-5}$ % à 0,5 % (pourcentages pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.10^{-5}$ % à 95 % (en poids).

Selon ce qui a déjà été dit les composés selon l'invention sont généralement associés à des supports et éventuellement des agents tensioactifs.

Par le terme « support », dans le présent exposé, on désigne une matière organique ou minérale,

naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides) ou liquide (eau, alcools, cétones, fractions de pétrole, hydrocarbures aromatiques ou paraffiniques, hydrocarbures chlorés, gaz liquéfiés).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénylsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions ; ces compositions selon l'invention sont elles-mêmes sous des formes assez diverses, solides ou liquides.

Comme formes de compositions solides, on peut citer les poudres pour poudrage ou dispersion (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 1 et 80 % pour ces derniers cas).

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,01 à 20 % de matière active. En plus du solvant, les concentrés émulsionnables peuvent contenir, quand c'est nécessaire, 2 à 20 % d'additifs appropriés, comme des stabilisants, des agents tensioactifs, des agents de pénétration, des inhibiteurs de corrosion, des colorants, des adhésifs. A titre d'exemple, voici la composition de quelques concentrés :

| | |
|---|---|
| matière active | 400 g/l |
| dodécylbenzène sulfonate alcalin | 24 g/l |
| nonylphénol oxyéthylé à 10 molécules d'oxyde d'éthylène | 16 g/l |
| cyclohexanone | 200 g/l |
| solvant aromatique | q.s.p 1 litre |

Selon une autre formule de concentré émulsionnable, on utilise :

| | |
|---|---|
| matière active | 250 g |
| huile végétale époxydée | 25 g |
| mélange de sulfonate d'alcoylaryle et d'éther de polyglycol et d'alcools gras | 100 g |
| diméthylformamide | 50 g |
| xylène | 575 g |

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide oganique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, des agents antimottants ou colorants.

A titre d'exemple, voici diverses compositions de poudres mouillables :

| | |
|---|---|
| matière active | 50 % |
| lignosulfonate de calcium (défloculant) | 5 % |
| isopropylnaphtalène sulfonate (agent mouillant anionique) | 1 % |

| | |
|---|---|
| silice antimottante | 5 % |
| kaolin (charge) | 39 % |

Poudre mouillable à 70 %:

| | |
|---|---|
| matière active | 700 g |
| dibutylnaphtylsulfonate de sodium | 50 g |
| produit de condensation en proportions 3/2/l d'acide naphtalène sulfonique, d'acide phénylsulfonique et de formaldéhyde | 30 g |
| kaolin | 100 g |
| craie de champagne | 120 g |

Poudre mouillable à 40 % :

| | |
|---|---|
| matière active | 400 g |
| lignosulfonate de sodium | 50 g |
| dibutylnaphtalène sulfonate de sodium | 10 g |
| silice | 540 g |

Poudre mouillable à 25 % :

| | |
|---|---|
| matière active | 250 g |
| lignosulfonate de calcium | 45 g |
| mélange équipondéral de craie de Champagne et d'hydroxyéthylcellulose | 19 g |
| dibutylnaphtalène sulfonate de sodium | 15 g |
| silice | 195 g |
| craie de Champagne | 195 g |
| kaolin | 281 g |

Poudre mouillable à 25 % :

| | |
|---|---|
| matière active | 250 g |
| isooctylphénoxy-polyoxyéthylène-éthanol | 25 g |
| mélange équipondéral de craie de Champagne et d'hydroxyéthylcellulose | 17 g |
| aluminosilicate de sodium | 543 g |
| kieselguhr | 165 g |

Poudre mouillable à 10 %

| | |
|---|---|
| matière active | 100 g |
| mélange de sels de sodium de sulfates d'acides gras saturés | 30 g |
| produit de condensation d'acide naphtalène sulfonique et de formaldéhyde | 50 g |
| kaolin | 820 g |

Les poudres solubles dans l'eau sont obtenues de manière usuelle en mélangeant de 20 à 95 % en poids de la matière active, de 0 à 10 % d'une charge antimottante, le reste étant constitué par un support solide hydrosoluble notamment un sel.

Voici un exemple de composition de poudre soluble :

| | |
|---|---|
| matière active (composé n° 2) | 70 % |
| agent mouillant anionique | 0,5 % |
| silice antimottante | 5 % |
| sulfate de sodium (support solide) | 24,5 % |

Pour obtenir ces poudres mouillables ou solubles, on mélange intimement les matières actives dans des mélangeurs appropriés avec les substances additionnelles et on broie avec des moulins ou autres broyeurs appropriés. On obtient par là des poudres dont la mouillabilité et la mise en suspension sont avantageuses ; on peut les mettre en suspension ou en solution dans l'eau à toute concentration désirée et cette suspension est utilisable très avantageusement en particulier pour l'application sur les feuilles des végétaux.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une « mayonnaise ».

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Selon un exemple de composition de granulé, on utilise les constituants suivants :

| | |
|---|---|
| matière active | 50 g |
| épichlorhydrine | 2,5 g |
| éther de cétyle et de polyglycol | 2,5 g |
| polyéthylène glycol | 35 g |
| kaolin (granulométrie : 0,3 à 0,8 mm) | 910 g |

Dans ce cas particulier on mélange la matière active avec l'épichlorhydrine et on dissout avec 60 g d'acétone ; on ajoute alors le polyéthylène glycol et l'éther de cétyle et de polyglycol. On arrose le kaolin avec la solution obtenue et on évapore ensuite l'acétone sous vide. On utilise avantageusement un tel microgranulé pour lutter contre les champignons du sol.

Les composés de formule (I) peuvent encore être utilisés sous forme de poudres pour poudrage : on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

**Revendications** (pour les Etats contractants : BE, CH, DE, FR, GB, IT, LI, LU, NL, SE)

1. Sel de dérivé organophosphoré caractérisé en ce qu'il répond à la formule :

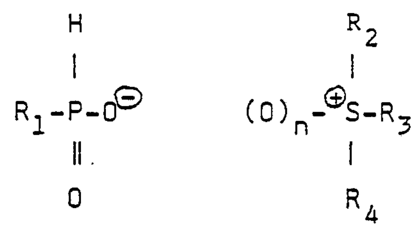

(I)

dans laquelle :

$R_1$ représente un atome d'hydrogène, un groupe hydroxyle, un radical alcoyle inférieur contenant de 1 à 4 atomes de carbone, éventuellement substitué par un atome d'halogène, un groupe hydroxyle ou hydroxylamino, ou représente un phényle, ou encore un radical $OR_5$, dans lequel $R_5$ représente un alcoyle contenant de 1 à 4 atomes de carbone,

$R_2$ et $R_3$, identiques ou différents, peuvent représenter un radical alcoyle contenant de 1 à 5 atomes de carbone, ou phényle, $R_2$ et $R_3$ pouvant en outre former ensemble un radical $-(CH_2)-_m$, m étant un nombre entier égal à 4 ou 5,

$R_4$ représente un radical alcoyle contenant de 1 à 18 atomes de carbone, un radical alcényle contenant de 2 à 18 atomes de carbone, le radical benzyle ou phényle,

n est un nombre entier égal à zéro ou 1.

2. Dérivé selon la revendication 1, caractérisé en ce que dans la formule, $R_1$ est un atome d'hydrogène, un radical hydroxyle ou alcoyle de 1 à 4 atomes de carbone, ou alcoxyle de 1 à 4 atomes de carbone, ou phényle, $R_2$, et $R_3$, sont chacun un radical alcoyle de 1 à 4 atomes de carbone notamment méthyle et éthyle, et $R_4$ est un radical méthyle ou un alcoyle linéaire de 12 à 16 atomes de carbone.

3. Dérivé selon la revendication 1, caractérisé en ce que dans la formule, $R_1$ est un radical hydroxyle ou alcoyle de 1 à 4 atomes de carbone, ou alcoxyle de 1 à 4 atomes de carbone, et $R_2$, $R_3$, $R_4$ sont chacun un radical méthyle.

4. Dérivé selon l'une des revendications 3 et 4, caractérisé en ce que, dans la formule, $R_1$ est le radical éthyle.

5. Procédé de préparation d'un composé selon l'une des revendications 1 à 4, caractérisé en ce qu'on effectue, en milieu aqueux, la réaction selon le schéma :

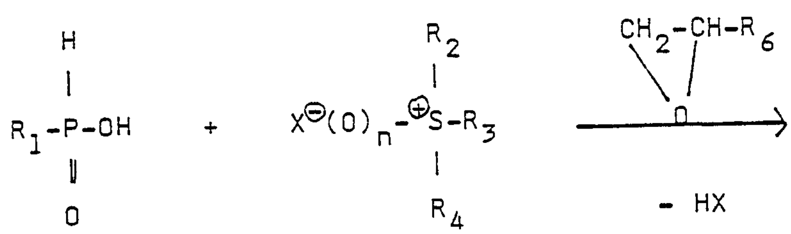


(I)

dans lequel $R_1$, $R_2$, $R_3$ et $R_4$ ont les mêmes significations qu'indiquées à la revendication 1, $R_6$ est un atome d'hydrogène ou un radical méthyle, et X est un atome de chlore, de brome ou d'iode, en présence d'un accepteur d'hydracide.

6. Procédé selon la revendication 5, caractérisé en ce que, pour les composés dans la formule desquels $R_1$ est le radical $OR_5$, l'ester de l'acide phosphoreux est préparé selon le schéma suivant :

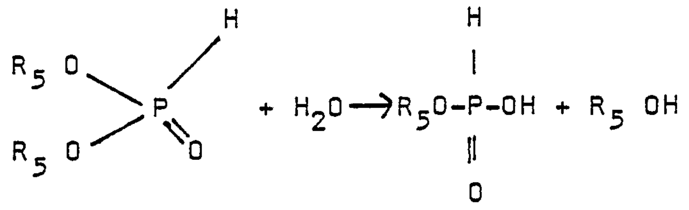

7. Procédé de préparation de composés selon les revendications 1 à 4, caractérisé en ce qu'on fait réagir un sulfate de sulfo(xo)nium sur un sel de calcium ou de baryum, selon le schéma :

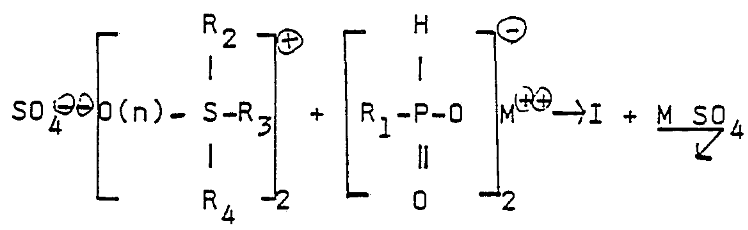

8. Compositions fongicides et/ou bactéricides à usage agricole caractérisées en ce qu'elles contiennent, comme matière active, un composé selon l'une des revendications 1 à 4.

9. Procédé de traitement de plantes contre les maladies, caractérisé en ce qu'on applique une composition selon la revendication 8.

**Revendications** (pour l'Etat contractant AT)

1. Composition fongicide et/ou bactéricide à usage agricole caractérisée en ce qu'elle contient, comme matière active, un composé de formule :

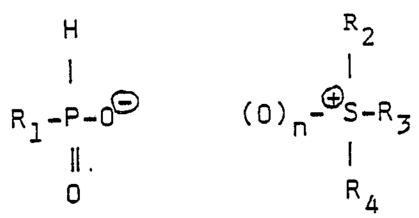

(I)

dans laquelle :

$R_1$ représente un atome d'hydrogène, un groupe hydroxyle, un radical alcoyle inférieur contenant de 1 à 4 atomes de carbone, éventuellement substitué par un atome d'halogène, un groupe hydroxyle ou hydroxylamino, ou représente un phényle, ou encore un radical $OR_5$, dans lequel $R_5$ représente un alcoyle contenant de 1 à 4 atomes de carbone,

$R_2$ et $R_3$, identiques ou différents, peuvent représenter un radical alcoyle contenant de 1 à 5 atomes de carbone, ou phényle, $R_2$ et $R_3$ pouvant en outre former ensemble un radical $-(CH_2)-_m$, m étant un nombre entier égal à 4 ou 5,

$R_4$ représente un radical alcoyle contenant de 1 à 18 atomes de carbone, un radical alcényle contenant de 2 à 18 atomes de carbone, le radical benzyle ou phényle,

n est un nombre entier égal à zéro ou 1.

2. Composition selon la revendication 1) caractérisée en ce que, dans la formule, $R_1$ est un atome d'hydrogène, un radical hydroxyle ou alcoyle de 1 à 4 atomes de carbone, ou alcoxyle de 1 à 4 atomes de carbone, ou phényle, $R_2$, et $R_3$ sont chacun un radical alcoyle de 1 à 4 atomes de carbone notamment méthyle et éthyle, et $R_4$ est un radical méthyle ou un alcoyle linéaire de 12 à 16 atomes de carbone.

3. Composition selon la revendication 1, caractérisée en ce que, dans la formule, $R_1$ est un radical hydroxyle ou alcoyle de 1 à 4 atomes de carbone, ou alcoxyle de 1 à 4 atomes de carbone, et $R_2$, $R_3$, $R_4$ sont chacun un radical méthyle.

4. Composition selon l'une des revendications 1 à 3, caractérisée en ce que, dans la formule, $R_1$ est le radical éthyle.

5. Procédé de traitement de plantes contre les maladies, caractérisé en ce qu'on applique une composition selon l'une des revendications 1 à 4.

6. Procédé de préparation d'un composé de formule :

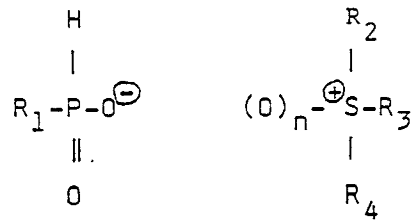

(I)

dans laquelle : $R_1$, $R_2$, $R_3$ et $R_4$ ont les mêmes significations qu'indiquées à la revendication 1, caractérisé en ce qu'on effectue, en milieu aqueux, la réaction selon le schéma :

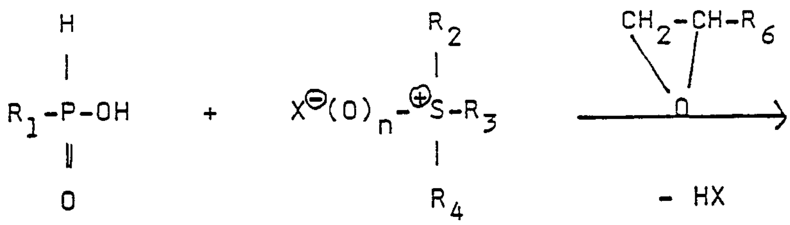


(I)

dans lequel $R_6$ est un atome d'hydrogène ou un radical méthyle, et X est un atome de chlore, de brome ou d'iode, en présence d'un accepteur d'hydracide.

7. Procédé selon la revendication 6, caractérisé en ce que, pour les composés dans la formule desquels $R_1$ est le radical $OR_5$, l'ester de l'acide phosphoreux est préparé selon le schéma suivant :

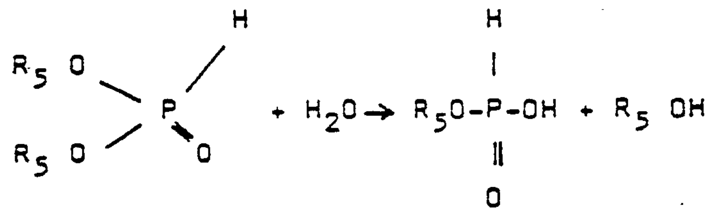

8. Procédé de préparation de composés de formule :

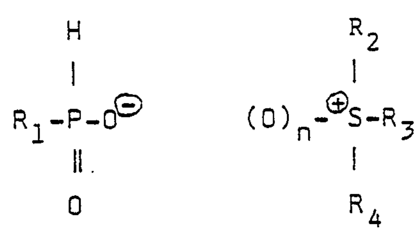

(I)

dans laquelle $R_1$, $R_2$, $R_3$ et $R_4$ ont les mêmes significations que celles indiquées à la revendication 1, caractérisé en ce qu'on fait réagir un sulfate de sulfo(xo)nium sur un sel de calcium ou de baryum, selon le schéma

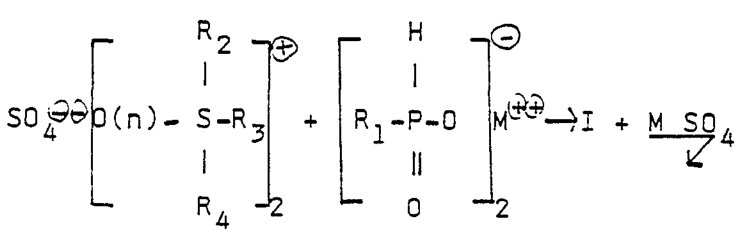

**Claims** (for the Contracting States : BE, CH, DE, FR, GB, IT, LI, LU, NL, SE)

1. A salt of an organophosphorus derivative, of the formula :

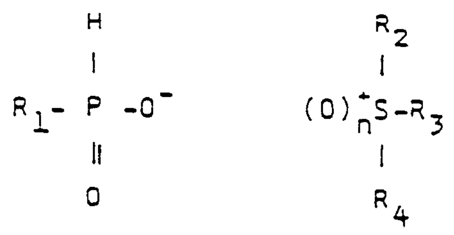

(I)

in which :

$R_1$ represents a hydrogen atom, a hydroxyl group, a lower alkyl radical containing from 1 to 4 carbon atoms, which is optionally substituted by a halogen atom, a hydroxyl or hydroxylamino group, an phenyl group or a radical $OR_5$, in which $R_5$ represents an alkyl containing from 1 to 4 carbon atoms,

$R_2$ and $R_3$, which are identical or different, can represent an alkyl radical containing from 1 to 5 carbon atoms or phenyl radical, it also being possible for $R_2$ and $R_3$ together to form a radical $-(CH_2)-_m$, m being an integer equal to 4 or 5,

$R_4$ represent an alkyl radical containing from 2 to 18 carbon atoms or the benzyl or phenyl radical, and

n is an integer equal to zero or 1.

2. A derivative according to claim 1, in the formula of which $R_1$ is a hydrogen atom, a hydroxyl radical, an alkyl radical having 1 to 4 carbon atoms, an alkoxy radical having 1 to 4 carbon atoms or a phenyl radical, $R_2$ and $R_3$ are each an alkyl radical having 1 to 4 carbon atoms, in particular methyl and ethyl, and $R_4$ is a methyl radical or a linear alkyl having 12 to 16 carbon atoms.

3. A derivative according to claim 1, in the formula of which $R_1$ is a hydroxyl radical, an alkyl radical having 1 to 4 carbon atoms or an alkoxy radical having 1 to 4 carbon atoms, and $R_2$, $R_3$ and $R_4$ are each a methyl radical.

4. A derivative according to one of claims 2 and 3, in the formula of which $R_1$ is the ethyl radical.

5. A process for the preparation of a compound according to one of claims 1 to 4, which comprises carrying out, in a aqueous medium, the reaction according to the equation :

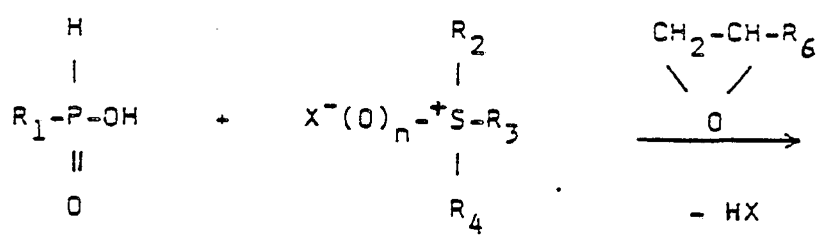

(I)

in which $R_1$, $R_2$, $R_3$ and $R_4$ have the same meanings as indicated in claim 1, $R_6$ is a hydrogen atom or a methyl radical and X is a chlorine, bromine or iodine atom, in the presence of a hydracid acceptor.

6. The process according to claim 5, wherein, for the compounds in the formula of which R-1 is the radical $OR_5$, the phosphorous acid ester is prepared according to the following equation :

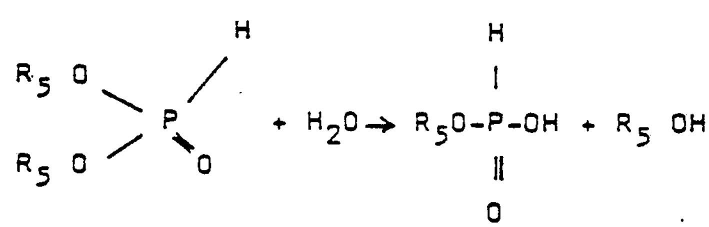

7. A process for the preparation of a compound according to claims 1 to 4, which comprises reacting a sulpho(xo)nium sulphate with a calcium or barium salt according to the equation :

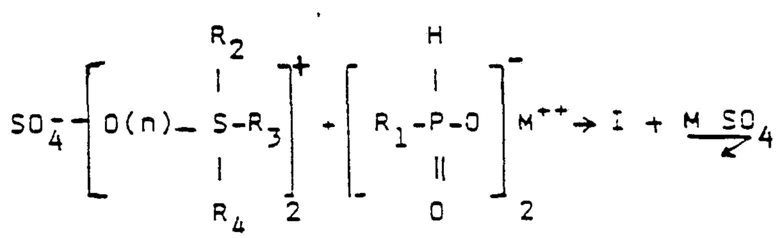

8. A fungicidal and/or bactericidal composition for agricultural use, which contains a compound according to one of claims 1 to 4 as the active ingredient.

9. A process for treating plants against diseases, which comprises applying a composition according to claim 8.

**Claims** (for the Contracting State AT)

1. A fungicidal and/or bactericidal composition for agricultural use, which contains a compound of formula :

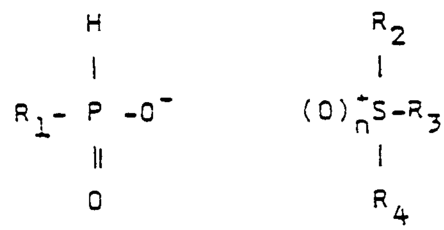

(I)

in which :

$R_1$ represents a hydrogen atom, a hydroxyl group, a lower alkyl radical containing from 1 to 4 carbon atoms, which is optionally substituted by a halogen atom, a hydroxyl or hydroxylamino group, an phenyl group or a radical $OR_5$, in which $R_5$ represents an alkyl containing from 1 to 4 carbon atoms,

$R_2$ and $R_3$, which are identical or different, can represent an alkyl radical containing from 1 to 5 carbon atoms or phenyl radical, it also being possible for $R_2$ and $R_3$ together to form a radical $-(CH_2)-_m$, m being an integer equal to 4 or 5,

$R_4$ represent an alkyl radical containing from 1 to 18 carbon atoms, an alkenyl radical containing from 2 to 18 carbon atoms or the benzyl or phenyl radical, and

n is an integer equal to zero or 1.

2. A composition according to claim 1, in the formula of which $R_1$ is a hydrogen atom, a hydroxyl radical, an alkyl radical having 1 to 4 carbon atoms, an alkoxy radical having 1 to 4 carbon atoms or a phenyl radical, $R_2$ and $R_3$ are each an alkyl radical having 1 to 4 carbon atoms, in particular methyl and ethyl, and $R_4$ is a methyl radical or a linear alkyl having 12 to 16 carbon atoms.

3. A composition according to claim 1, the formula of which $R_1$ is a hydroxyl radical, an alkyl radical having 1 to 4 carbon atoms or an alkoxy radical having 1 to 4 carbon atoms, and $R_2$, $R_3$ and $R_4$ are each a methyl radical.

4. A composition according to one of claims 2 and 3, in the formula of which $R_1$ is the ethyl radical.

5. A process for treating plants against diseases, which comprises applying a composition according to claims 1 to 4.

6. A process for the preparation of a compound of the formula

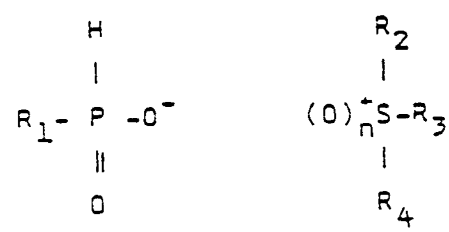

(I)

wherein $R_1$, $R_2$, $R_3$ and $R_4$ have the same meanings as in claim 1, which comprises carrying out, in a aqueous medium, the reaction according to the equation :

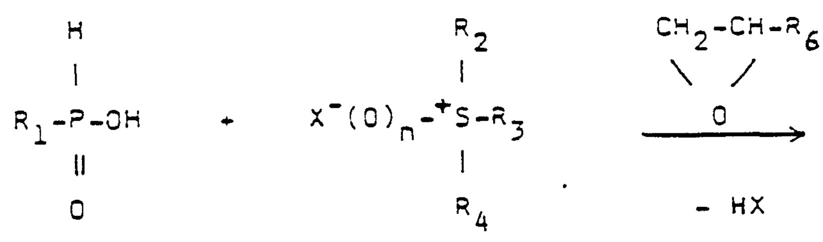


(I)

in which $R_1$, $R_2$, $R_3$ and $R_4$ have the same meanings as indicated in claim 1, $R_6$ is a hydrogen atom or a methyl radical and X is a chlorine, bromine or iodine atom, in the presence of a hydracid acceptor.

7. The process according to claim 6, wherein, for the compounds in the formula of which $R_1$ is the radical $OR_5$, the phosphorous acid ester is prepared according to the following equation :

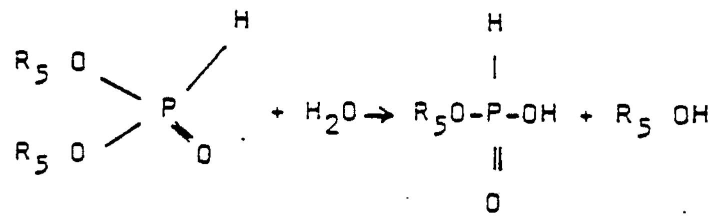

8. A process for the preparation of a compound according to claims 1 to 4, which comprises reacting a sulpho(xo)nium sulphate with a calcium or barium salt according to the equation :

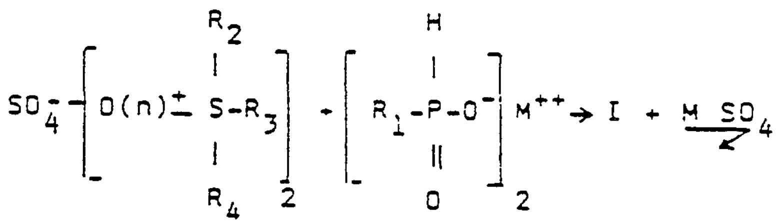

**Patentansprüche** (für die Vertragsstaaten : BE, CH, DE, FR, GB, IT, LI, LU, NL, SE)

1. Salze von Organophosphorderivaten, gekennzeichnet durch die Formel I

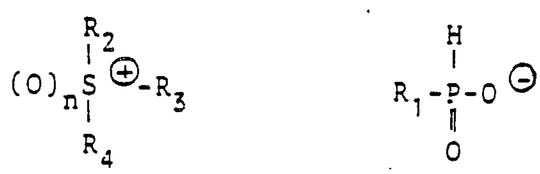

in der bedeuten :

$R_1$ Wasserstoff, Hydroxy, $C_1$- bis $C_4$-Alkyl, das gegebenenfalls mit einem Halogenatom, einer Hydroxygruppe oder einer Hydroxylaminogruppe substituiert ist, Phenyl, oder eine Gruppe $—OR_5$, in der $R_5$ $C_1$- bis $C_4$-Alkyl bedeutet,

$R_2$ und $R_3$ zugleich oder unabhängig $C_1$- bis $C_5$-Alkyl oder Phenyl, wobei $R_2$ und $R_3$ auch zusammen eine Gruppe $—(CH_2)_m—$ bilden können, bei der m 4 oder 5 bedeutet,

$R_4$ $C_1$- bis $C_{18}$-Alkyl, $C_2$- bis $C_{18}$-Alkenyl, Benzyl oder Phenyl ist, und

n 0 oder 1

2. Salze der Formel I nach Anspruch 1, dadurch gekennzeichnet, daß $R_1$ Wasserstoff, Hydroxy, $C_1$- bis $C_4$-Alkyl, $C_1$- bis $C_4$-Alkoxy oder Phenyl, $R_2$ und $R_3$ jeweils $C_1$- bis $C_4$-Alkyl und $R_4$ Methyl oder geradkettiges $C_{12}$- bis $C_{16}$-Alkyl bedeuten.

3. Salze der Formel I nach einem der Ansprüche 1 und 2, dadurch gekennzeichnet, daß $R_1$ Hydroxy, $C_1$- bis $C_4$-Alkyl oder $C_1$- bis $C_4$-Alkoxy und $R_2$, $R_3$ und $R_4$ jeweils Methyl bedeuten.

4. Salze der Formel I nach einem der Ansprüche 1 bis 3, dadurch gekennzeichnet, daß $R_1$ Ethyl bedeutet.

5. Verfahren zur Herstellung der Salze nach einem der Ansprüche 1 bis 4, gekennzeichnet durch Umsetzung nach folgendem Reaktionsschema :

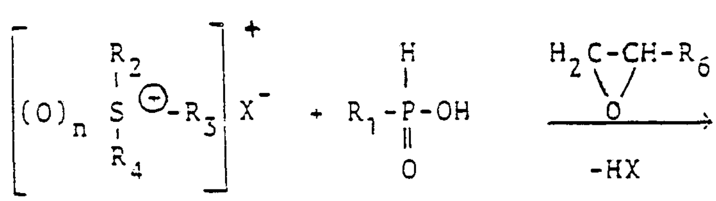


(I)

wobei bedeuten :

$R_1$, $R_2$, $R_3$, $R_4$ und n dasselbe wie in Anspruch 1,

$R_6$ Wasserstoff oder Methyl und

X Chlor, Brom oder Jod, in wäßrigem Medium in Gegenwart eines Säureakzeptors.

6. Verfahren nach Anspruch 5, dadurch gekennzeichnet, daß monosubstituierte Phosphite mit $R_1 = OR_5$ mit $R_5$ wie in Anspruch 1 eingesetzt werden, die durch Hydrolyse des entsprechenden Dialkylphosphits nach dem Reaktionsschema

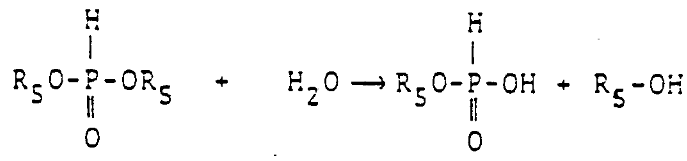

hergestellt werden.

7. Verfahren zur Herstellung der Salze nach einem der Ansprüche 1 bis 4, gekennzeichnet durch Umsetzung eines Calcium- oder Bariumsalzes einer substituierten Phosphorverbindung mit einem Sulfonium- bzw. Sulfoxoniumsulfat nach folgendem Reaktionsschema :

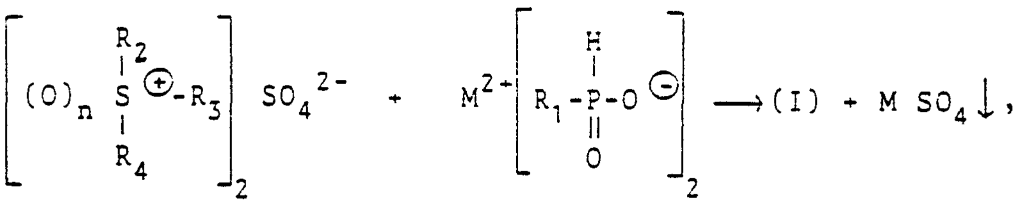

worin $R_1$, $R_2$, $R_3$, $R_4$ und n die Bedeutung von Anspruch 1 bzw. 6 besitzen und M Calcium oder Barium bedeutet.

8. Fungicide und/oder bactericide Zusammensetzungen zum Pflanzenschutz, gekennzeichnet durch mindestens eine Verbindung nach einem der Ansprüche 1 bis 4 als Wirkstoff.

9. Verfahren zur Behandlung von Pflanzen gegen Pilz- und/oder Bakterienerkrankungen, gekennzeichnet durch Anwendung einer Zusammensetzung nach Anspruch 8.

**Patentansprüche** (für den Vertragsstaat AT)

1. Fungicide und/oder bactericide Zusammensetzungen zum Pflanzenschutz, gekennzeichnet durch mindestens einem Salz von Organophosphorderivaten der Formel I

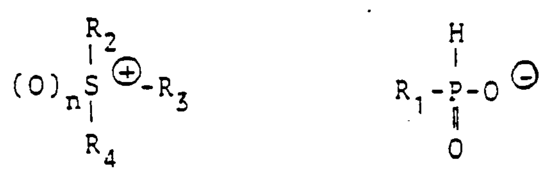

(I)

in der bedeuten :

$R_1$ Wasserstoff, Hydroxy, $C_1$- bis $C_4$-Alkyl, das gegebenenfalls mit einem Halogenatom, einer Hydroxygruppe oder einer Hydroxylaminogruppe substituiert ist, Phenyl, oder eine Gruppe $-OR_5$, in der $R_5$ $C_1$- bis $C_4$-Alkyl bedeutet,

$R_2$ und $R_3$ zugleich oder unabhängig $C_1$- bis $C_5$-Alkyl oder Phenyl, wobei $R_2$ und $R_3$ auch zusammen eine Gruppe $-(CH_2)_m-$ bilden können, bei der m 4 oder 5 bedeutet,

$R_4$ $C_1$- bis $C_{18}$-Alkyl, $C_2$- bis $C_{18}$-Alkenyl, Benzyl oder Phenyl,

und

n 0 oder 1,

als Wirkstoff.

2. Zusammensetzungen nach Anspruch 1, dadurch gekennzeichnet, daß $R_1$ Wasserstoff, Hydroxy, $C_1$- bis $C_4$-Alkyl, $C_1$- bis $C_4$-Alkoxy oder Phenyl, $R_2$ und $R_3$ jeweils $C_1$- bis $C_4$-Alkyl und $R_4$ Methyl oder geradkettiges $C_{12}$- bis $C_{16}$-Alkyl bedeuten.

3. Zusammensetzungen nach einem der Ansprüche 1 und 2, dadurch gekennzeichnet, daß $R_1$ Hydroxy, $C_1$- bis $C_4$-Alkyl oder $C_1$- bis $C_4$-Alkoxy und $R_2$, $R_3$ und $R_4$ jeweils Methyl bedeuten.

4. Zusammensetzungen nach einem der Ansprüche 1 bis 3, dadurch gekennzeichnet, daß $R_1$ Ethyl bedeutet.

5. Verfahren zur Behandlung von Pflanzen gegen Pilz- und/oder Bakterienerkrankungen, gekennzeichnet durch Anwendung einer Zusammensetzung nach einem der Ansprüche 1 bis 4.

6. Verfahren zur Herstellung einer Verbindung der Formel I

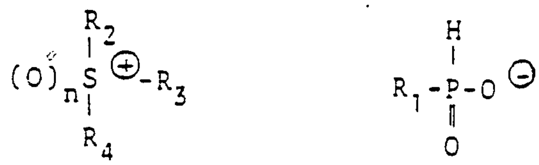

(I)

in der $R_1$, $R_2$, $R_3$ und $R_4$ dieselbe Bedeutungen als in Anspruch 1 haben, gekennzeichnet durch Umsetzung nach folgendem Reaktionsschema :

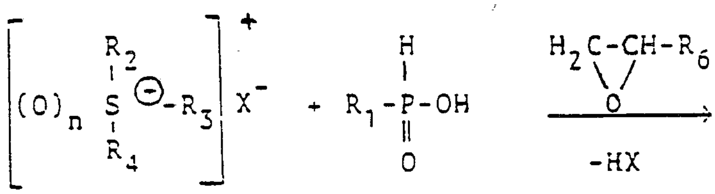


(I)

wobei bedeuten :

$R_1$, $R_2$, $R_3$, $R_4$ und n dasselbe wie in Anspruch 1,
$R_6$ Wasserstoff oder Methyl und
X Chlor, Brom oder Jod,

in wäßrigem Medium in Gegenwart eines Säureakzeptors.

7. Verfahren nach Anspruch 6, dadurch gekennzeichnet, daß monosubstituierte Phosphite mit $R_1 = OR_5$ mit $R_5$ wie in Anspruch 1 eingesetzt werden, die durch Hydrolyse des entsprechenden Dialkylphosphits nach dem Reaktionsschema

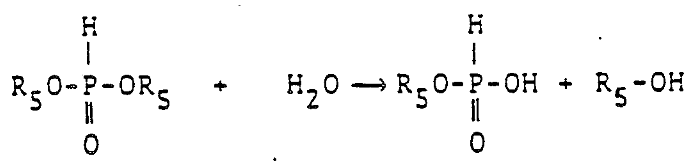

hergestellt werden.

8. Verfahren zur Herstellung einer Verbindung der Formel I

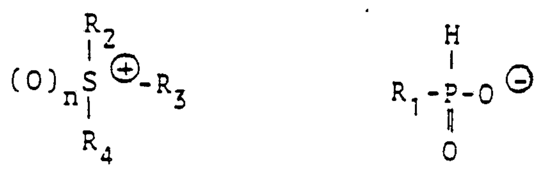

(I)

in der $R_1$, $R_2$, $R_3$ und $R_4$ dieselbe Bedeutungen als in Anspruch 1 haben, gekennzeichnet durch Umsetzung eines Calcium- oder Bariumsalzes einer substituierten Phosphorverbindung mit einem Sulfonium- bzw. Sulfoxoniumsulfat nach folgendem Reaktionsschema :

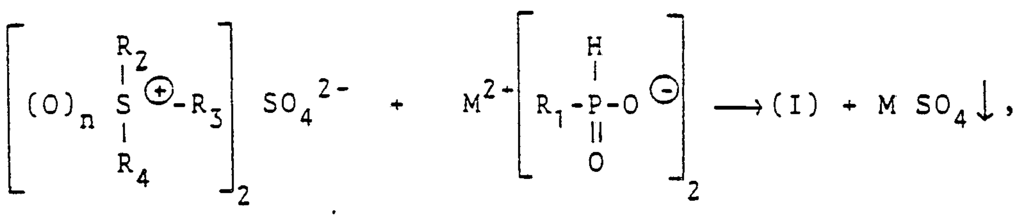

worin $R_1$, $R_2$, $R_3$, $R_4$ und n die Bedeutung von Anspruch 1 bzw. 4 besitzen und M Calcium oder Barium bedeutet.